(19) Europäisches Patentamt
European Patent Office
Office européen des brevets

(11) Veröffentlichungsnummer : **0 043 031**
**B2**

(12) # NEUE EUROPÄISCHE PATENTSCHRIFT

(45) Veröffentlichungstag der neuen Patentschrift : **13.04.88**

(51) Int. Cl.⁴ : **C 07 C 85/26**, C 07 C 93/14, C 07 C 87/60

(21) Anmeldenummer : **81104642.4**

(22) Anmeldetag : **16.06.81**

(54) **Verfahren zur Gewinnung von reinen Anilin-Verbindungen.**

(30) Priorität : **26.06.80 DE 3023883**

(43) Veröffentlichungstag der Anmeldung : **06.01.82 Patentblatt 82/01**

(45) Bekanntmachung des Hinweises auf die Patenterteilung : **27.07.83 Patentblatt 83/30**

(45) Bekanntmachung des Hinweises auf die Entscheidung über den Einspruch : **13.04.88 Patentblatt 88/15**

(84) Benannte Vertragsstaaten : **CH DE FR GB IT LI**

(56) Entgegenhaltungen :
**DE-C- 944 952**
**US-A- 3 510 266**
**Bios Final Report, Nr. 986, S. 282-284 und 288-290**
**Chem. and Ind., 1973, S. 139-142**
**Fortschritte der Verfahrenstechnik, Bd. 12 (1973)/74, S. 334**
**Chemie-Ing.-Techn. 45. Jahrg. (1973), S. 136-138**
**Verfahrenstechn. Berichte, ISSN 0042-3890, Nr. 7323/22 u. 7450/2**
**Ullmanns Enzyklopaedie, 4. Auflage, Bd. 2, S. 679**
**Chem. Abstr., vol. 71 (1969), 123907z**
**Chem. Abstr., vol. 73 (1970), 26585b**
**Chem. Abstr., vol. 82 (1975), 60590v**

**Die Akte enthält technische Angaben, die nach dem Eingang der Anmeldung eingereicht wurden und die nicht in dieser Patentschrift enthalten sind.**

(73) Patentinhaber : **HOECHST AKTIENGESELLSCHAFT**
**Postfach 80 03 20**
**D-6230 Frankfurt am Main 80 (DE)**

(72) Erfinder : **Papenfuhs, Theodor, Dr.**
**Heinrich-Bleicher-Strasse 40**
**D-6000 Frankfurt am Main 50 (DE)**

EP 0 043 031 B2

**Beschreibung**

Die Erfindung liegt auf dem technischen Gebiet der Zwischenprodukte und betrifft ein verbessertes Verfahren zur Gewinnung von Anilin-Verbindungen in reiner Form.

Primäre aromatische Amine, insbesondere der Benzolreihe, die keine wasserlöslich machende Gruppe besitzen, dienen als wertvolle Diazokomponenten zur Erzeugung von wasserunlöslichen Azofarbstoffen auf der Faser nach den Methoden der Eisfarbentechnik. Hierunter sind auch solche primäre Anilinderivate von besonderem technischen Interesse, die durch elektronegative Substituenten substituiert sind, wie beispielsweise 1-Amino-2-methoxy-4-nitrobenzol (C.I.-Nr. 37 125), 1-Amino-2-methoxy-5-nitrobenzol (C.I.-Nr. 37 130) und 1-Amino-2-nitro-4-methoxybenzol (C.I.-Nr. 37 135). Solche negativ substituierte Amine haben beispielsweise auch erhebliche technische Bedeutung als Farbstoffzwischenprodukte. Für diesen Verwendungszweck, insbesondere in der Eisfarbentechnik, müssen die aromatischen Amine ein hohes Maß an Reinheit aufweisen, so daß sich an ihre Synthese im allgemeinen ein Reinigungsverfahren anschließt. Die bisher bekannten Verfahren erfordern diesbezüglich aber hohe Investitionen und einen hohen Wasser- und Energiebedarf. So erhält man beispielsweise Nitro-methoxyanilin-Verbindungen aus deren Synthese oft mit Isomeren verbunden und ausnahmslos mit harzigen Produkten verunreinigt; mit einer technisch sehr aufwendigen Umlösung unter Druck aus sehr verdünnten, 1-2 %igen, gegebenenfalls sauren, wäßrigen Medien führt man die gewünschten Anilinderivate zu reinen Produkten (vgl. BIOS FINAL REPORT Nr. 986, Seiten 282-284 und 288-290).

Es hat nicht an Versuchen gefehlt, effektivere Reinigungsmethoden zu entwickeln. Gemessen am Apparate- und Energieaufwand boten hierbei Umkristallisationen aus Lösemitteln die größte Aussicht auf Erfolg. In keinem Falle ist es aber bisher gelungen, durch Umkristallisation diese primären aromatischen Amine auf wirtschaftliche Weise in der geforderten hohen Reinheit zu erhalten. Hohe Lösungsmittelverluste bei nur mäßigem Reinigungseffekt, beispielsweise infolge Einschlusses von Verunreinigungen bei der Kristallisation, machten die Verfahren technisch uninteressant.

Mit der vorliegenden Erfindung wurde nunmehr ein technisch anwendbares Verfahren zur Gewinnung von reinen Anilin-Verbindungen, mit elektronegativen Substituenten, jedoch keinen wasserlöslichmachenden Substituenten, gefunden. Dieses neue Reinigungsverfahren gestattet es, die genannten Aniline bei lediglich geringem Bedarf und Aufwand an Energie und Apparaturen technisch ohne nennenswerten Lösemittelverlust in ausgezeichneter Reinheit und Ausbeute zu gewinnen. Das erfindungsgemäße Verfahren ist dadurch gekennzeichnet, daß man die verunreinigten Anilin-Verbindungen mit elektronegativen Substituenten in einem Alkanol mit 1 bis 5 C-Atomen in der Wärme löst, diese Lösung einer Klärfiltration zuführt, das Filtrat während oder nach dessen Abkühlung einem Naßzerkleinerungsprozeß unterwirft, anschließend die Anilin-Verbindung durch Filtration isoliert und das hieraus erhaltene Filtrat (Mutterlauge) nach Lösung einer weiteren Menge der verunreinigten Anilin-Verbindung in der Wärme dem Reinigungsverfahren zuführt, das Reinigungsverfahren in der angegebenen Weise durchführt und diesen Prozeß der Recyclisierung der Mutterlauge und des Reinigungsverfahrens noch mindestens zehn Mal wiederholt.

Alkanole mit 1 bis 5 C-Atomen, in denen die Anilin-Verbindung in der Wärme gelöst wird, sind z. B. n-Butanol und insbesondere Methanol, Äthanol, Isopropanol und n-Propanol. Die Lösung der Anilin-Verbindungen, die gegebenenfalls wasserfeucht sein können, erfolgt unterhalb des Siedepunktes oder beim Siedepunkt des Lösungsmittels, vorzugsweise bei einer Temperatur oberhalb von 50 °C.

Bei der Klärfiltration dient als klärendes Agenz ein übliches Adsorbtionsmittel, wie beispielsweise Klärkohlen (Aktivkohlen) oder mineralische Adsorbentien, wie Kieselgur, Diatomeenerde oder Bentonite, oder Mischungen derselben, insbesondere mit Aktivkohle. In der für diese Reinigungsprozesse üblichen Weise kann man die Lösungen der Anilin-Verbindungen mit diesen Adsorbentien versetzen und in der Wärme behandeln und anschließend filtrieren oder aber die heißen Lösungen der Anilin-Verbindungen durch eine Schicht dieser Adsorbentien hindurchführen. Das heiße oder abgekühlte Filtrat wird dann einer in der Technik üblichen Naßmühle, beispielsweise einer Trichter-oder Kugelmühle, zugeführt und die während oder nach der Abkühlung des Lösemittels ausfallende Anilin-Verbindung in dem Lösemittel einem Naßzerkleinerungsprozeß in der Naßmühle unterworfen. Die hierbei anfallende feinteilige Suspension der Anilin-Verbindung wird anschließend filtriert; die so isolierte hochreine Anilin-Verbindung kann auf übliche Weise getrocknet werden.

Das nach Filtration der Suspension aus der Naßmühle erhaltene Filtrat (Mutterlauge) wird, wie oben angegeben, dem nächsten, erneuten, erfindungsgemäßen Reinigungsverfahren zugeführt. Je nach Verunreinigungsgrad der eingesetzten Anilin-Verbindung und nach Sättigung der Mutterlaugen in diesen Nebenprodukten kann die Recyclisierung etwa zehn bis vierzig Mal erfolgen. Das nach solchen Recyclisierungsverfahren mit den Verunreinigungen stark angereicherte Filtrat wird schließlich, gegebenenfalls nach fraktionierter Konzentrierung und teilweiser Abdestillation des Lösemittels, auf einem üblichen Wege, beispielsweise durch Verbrennen, entsorgt.

Elektronegative Substituenten der Anilin-Verbindungen sind insbesondere die Halogenatome, wie bevorzugt das Chlor- und Bromatom, die Nitrogruppe, die Cyangruppe, die Trifluormethylgruppe und die Carbonamid- und Sulfonamidgruppen. Die elektronegativ substituierten Aniline können auch zusätzlich andere, elektroneutrale und/oder elektropositive Substituenten, wie Methyl-, Äthyl-, Methoxy- und Äthoxygruppen, enthalten.

Das erfindungsgemäße Verfahren eignet sich besonders zur Reinigung von Nitranilin-, Nitroanisidin-, Nitrophenitidin-, Halogenanisidin-, Halogenphenetidin-, Halogennitranilin- und Halogennitrokresidin-Verbindungen, beispielsweise solchen der allgemeinen Formel (1)

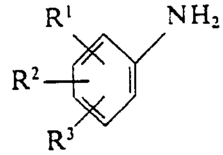

(1)

in welcher $R^1$ für ein Wasserstoffatom, die Methoxy-, Äthoxy-, Methyl- oder Äthylgruppe steht, $R^2$ die Nitrogruppe, ein Chlor- oder Bromatom oder die Cyan- oder Trifluormethylgruppe bedeutet und $R^3$ ein Wasserstoffatom, die Methoxy-, Äthoxy-, Methyl- oder Äthylgruppe oder ein Chlor- oder Bromatom darstellt, wobei $R^1$, $R^2$ und $R^3$ zueinander gleich oder voneinander verschiedene Bedeutungen besitzen können, insbesondere bevorzugt zur Reinigung von Verbindungen der allgemeinen Formel (1), in welcher $R^1$ ein Wasserstoffatom, $R^2$ die Nitrogruppe oder ein Chloratom und $R^3$ die Methoxy- oder Äthoxygruppe ist.

Mit dem erfindungsgemäßen Verfahren wird gegenüber bekannten Verfahren eine 10- bis 20fache Steigerung der Raum/Zeit-Ausbeute erreicht ; das erfindungsgemäße Verfahren führt auch weiterhin zu einer erheblichen Senkung des Energie- und Apparateaufwandes und liefert praktisch analysenreine Produkte. Es gestattet eine leichte technische Durchführung, gegebenenfalls auch in kontinuierlicher Arbeitsweise.

Die nachfolgenden Beispiele dienen zur Erläuterung der Erfindung. Die Teile sind Gewichtsteile, die Prozentangaben beziehen sich auf Gewichtsprozent, sofern nicht anders vermerkt.

## Beispiel 1

1 300 Teile Isopropanol wurden mit 265,1 Teilen wasserfeuchtem, rohem 1-Amino-2-nitro-4-methoxy-benzol, das aus der Synthese erhalten wurde und 4,2 % Verunreinigungen enthielt und einen Wassergehalt von 33,9 % besaß, sowie mit 22 Teilen Aktivkohle und 1,1 Teilen Natriumcarbonat versetzt. Diese Mischung wurde unter Rühren zum Sieden unter Rückfluß (mit einer Rückflußtemperatur von etwa 85 °C) erhitzt und noch 15 Minuten lang bei dieser Temperatur gehalten. Anschließend wurde dieser Ansatz über ein beheiztes Druckfilter geklärt und der Filterrückstand mit 35 Teilen Isopropanol nachgewaschen. Das vereinigte Filtrat wurde unter Rühren auf 10-15 °C abgekühlt und anschließend in einer Naßmühle gemahlen. Die erhaltene Suspension wurde filtriert, der Filterkuchen mit 35 Teilen Isopropanol und anschließend, nach Wechsel der Vorlagen, mit 500 Teilen Wasser gewaschen und getrocknet.

Das vereinigte Isopropanolfiltrat wird als Lösemittel für die nächste Reinigung gemäß der oben beschriebenen Verfahrensweise anstelle der 1 300 Teile Isopropanol eingesetzt. Das wäßrige Filtrat kann verworfen werden.

Es wurden 134,5 Teile analysenreines 1-Amino-2-nitro-4-methoxy-benzol entsprechend einer Ausbeute von 79,2 % d. Th. erhalten ; dieses analysenreine Produkt lieferte bei der Verwendung als Diazokomponente in der Eisfarbentechnik typkonforme Färbungen.

Entsprechend der hier angegebenen erfindungsgemäßen Verfahrensweise wurde die Reinigung des wasserfeuchten, rohen 1-Amino-2-nitro-4-methoxy-benzols noch 20mal durchgeführt, wobei in dem zweiten und in den nachfolgenden Ansätzen als Lösemittel nicht mehr frisches Isopropanol, sondern jeweils die aus dem vorigen Ansatz als Filtrat erhaltene isopropanolische Mutterlauge eingesetzt wurde ; im übrigen wurde in gleicher Arbeitsweise, wie oben beschrieben, die Reinigung und Isolierung des gereinigten Produktes durchgeführt. Nach dem 20. Ansatz war das isopropanolische Filtrat so stark verunreinigt, daß es nicht mehr weiterverwendet werden konnte ; es wurde durch Verbrennen vernichtet.

Bei dem 20fachen Recyclisierungsprozeß wurden jeweils im Mittel 156,7 Teile analysenreines, färberisch einwandfreies 1-Amino-2-nitro-4-methoxy-benzol erhalten, was pro Ansatz einer durchschnittlichen Ausbeute von 93,3 % d. Th. entspricht. Die geringere Ausbeute des ersten, oben beschriebenen Ansatzes fällt somit nicht ins Gewicht.

## Beispiele 2 bis 15

Man verfährt in erfindungsgemäßer Weise zur Gewinnung von wasserunlöslichen, negativ substituierten primären Aminen, beispielsweise analog der im Beispiel 1 angegebenen Methode, und setz hierbei die in den nachfolgenden Tabellenbeispielen durch die allgemeine Formel (1) charakterisierten Anilinderivate als Rohprodukte mit dem dort angegebenen Gehalt an Verunreinigungen und gegebenenfalls Wasser ein. Man erhält bei den in diesen Tabellenbeispielen angegebenen Reaktionsparametern in den angegebenen durchschnittlichen Ausbeuten analysenreine aromatische Amine, die beispielsweise in der Eisfarbentechnik eingesetzt werden können und dort typkonforme einwandfreie Färbungen liefern. Die in den Tabellenbeispielen angegebenen Mengen an Lösemitteln und Adsorbentien beziehen sich jeweils auf ein Mol Amin der Formel (1). Die Menge des Lösemittels bezieht sich auf den ersten Reinigungsansatz.

| Bsp. | Amin der Formel (1) $R^1$ | $R^2$ | $R^3$ | Wasser-geh. (%) | Verun-rein. (%) | Lösemittel | Absorbens | Temp. (°C) | Recycl. der Mut-terlauge | Durchschn. Ausbeute (% d. Th.) |
|---|---|---|---|---|---|---|---|---|---|---|
| 2 | 2-$OCH_3$ | 4-$NO_2$ | H | 34,5 | 1,6 | Isopropanol (1300 g) | A-Kohle (10 g) | 85 | 30 × | 92,2 |
| 3 | 2-$OCH_3$ | 4-$NO_2$ | H | 34,5 | 1,6 | Methanol (1400 g) | A-Kohle (10 g) | 70 | 25 × | 93,0 |
| 4 | 4-$OCH_3$ | 2-$NO_2$ | H | 33,9 | 4,2 | Methanol (1500 g) | A-Kohle (20 g) | 70 | 20 × | 94,1 |
| 5 | 4-$OCH_3$ | 2-$NO_2$ | H | 33,9 | 4,2 | Äthanol (1400 g) | A-Kohle (20 g) | 80 | 20 × | 93,8 |
| 6 | 4-$OCH_3$ | 2-$NO_2$ | H | – | 4,2 | Methanol (1100 g) | A-Kohle (20 g) | 70 | 30 × | 92,6 |
| 7 | 4-$OCH_3$ | 2-$NO_2$ | H | 33,9 | 4,2 | Methanol (1500 g) | Bleicherde (30 g) | 70 | 20 × | 93,7 |
| 8 | 4-$OCH_3$ | 2-$NO_2$ | H | 37,1 | 4,8 | iso-Butanol (800 g) | A-Kohle (30 g) | 90 | 15 × | 92,7 |
| 9 | H | 3-$NO_2$ | H | 34,3 | 3,0 | Isopropanol (950 g) | A-Kohle (10 g) | 85 | 30 × | 96,2 |

(Fortsetzung)

| Bsp. | Amin der Formel (1) $R^1$ | $R^2$ | $R^3$ | Wasser-geh. (%) | Verun-rein. (%) | Lösemittel | Absorbens | Temp. (°C) | Recycl. der Mut-terlauge | Durchschn. Ausbeute (% d. Th.) |
|---|---|---|---|---|---|---|---|---|---|---|
| 10 | H | 3-$NO_2$ | H | – | 3,0 | Äthanol (750 g) | A-Kohle (10 g) | 80 | 35 × | 95,8 |
| 11 | H | 3-$NO_2$ | H | 34,3 | 3,0 | Äthanol (900 g) | Bentonit (15 g) | 80 | 25 × | 96,0 |
| 12 | 2-$OCH_3$ | 4-Cl | 5-$OCH_3$ | 29,8 | 5,2 | Isopropanol (1250 g) | A-Kohle (25 g) | 85 | 20 × | 94,2 |
| 13 | 2-$OCH_3$ | 5-Cl | 4-$OCH_3$ | 37,2 | 6,8 | n-Butanol (1200 g) | A-Kohle (35 g) | 90 | 12 × | 91,4 |
| 14 | 2-$OCH_3$ | 4-Cl | 5-$CH_3$ | 30,4 | 4,1 | Äthanol (1200 g) | A-Kohle (20 g) | 80 | 15 × | 93,8 |
| 15 | 4-$OC_2H_5$ | 2-$NO_2$ | H | 32,8 | 5,0 | Isopropanol (1000 g) | A-Kohle (25 g) | 85 | 20 × | 92,7 |

**Patentansprüche**

1. Verfahren zur Gewinnung von reinen, elektronegativ substituierten Anilin-Verbindungen ohne wasserlöslichmachende Substituenten, dadurch gekennzeichnet, daß man die verunreinigte Anilin-Verbindung in einem Alkanol von 1 bis 5 C-Atomen in der Wärme löst, diese Lösung einer Klärfiltration zuführt, das Filtrat während oder nach dessen Abkühlung einem Naßzerkleinerungsprozeß unterwirft, anschließend die Anilin-Verbindung durch Filtration isoliert und das hieraus erhaltene Filtrat (Mutterlauge) nach Lösung einer weiteren Menge der verunreinigten Anilin-Verbindung in der Wärme dem Reinigungsverfahren zuführt, das Reinigungsverfahren in der angegebenen Weise durchführt und diesen Prozeß der Recyclisierung der Mutterlauge und des Reinigungsverfahrens noch mindestens zehn Mal wiederholt.

2. Verfahren nach Anspruch 1, dadurch gekennzeichnet, daß die elektronegativ substituierte Anilin-Verbindung der allgemeinen Formel (1)

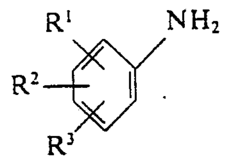

(1)

entspricht, in welcher $R^1$ für ein Wasserstoffatom, die Methoxy-, Äthoxy-, Methyl- oder Äthylgruppe steht, $R^2$ die Nitrogruppe, ein Chlor- oder Bromatom oder die Cyan- oder Trifluormethylgruppe bedeutet und $R^3$ ein Wasserstoffatom, die Methoxy-, Äthoxy-, Methyl- oder Äthylgruppe oder ein Chlor- oder Bromatom darstellt, wobei $R^1$, $R^2$ und $R^3$ zueinander gleich oder voneinander verschiedene Bedeutungen besitzen können.

**Claims**

1. Process for the preparation of pure, electronegatively substituted aniline compounds containing no watersolubilizing substituents, characterized in that the contaminated aniline compound it dissolved, by heat, in an alkanol with 1 to 5 C-atoms, that this solution is subjected to a clarifying filtration, that the filtrate is subjected, during or after its cooling, to a wet comminution process, that the aniline compound is then isolated by filtration and the filtrate (mother liquor) thereof obtained is conveyed, after dissolution of another amount of the contaminated aniline compound by heat, to the purification process, the purification process is carried out in the above-indicated manner, and this procedure of recycling the mother liquor and the purification process is repeated at least 10 times.

2. Process according to claim 1, characterized in that the electronegatively substituted aniline compound employed corresponds to the general formula (1)

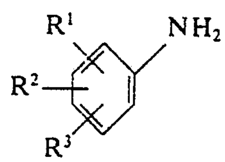

(1)

in which $R^1$ represents a hydrogen atom, the methoxy, ethoxy, methyl or ethyl group, $R^2$ means the nitro group, a chlorine or bromine atom or the cyano or trifluoromethyl group and $R^3$ represents a hydrogen atom, the methoxy, ethoxy, methyl or ethyl group or a chlorine or bromine atom, and $R^1$, $R^2$ and $R^3$ can have meanings which are identical to or different from one another.

**Revendications**

1. Procédé de préparation d'anilines pures porteuses de substituants électronégatifs mais dépourvues de substituants hydrosolubilisants, procédé caractérisé en ce qu'on dissout à chaud l'aniline impure dans un alcanol contenant de 1 à 5 atomes de carbone, on fait subir à cette solution une clarification par filtration, on soumet le filtrat, pendant ou après le refroidissement de celui-ci, à une opération de broyage par voie humide, puis on isole le dérivé de l'aniline par filtration, on envoie le filtrat ainsi obtenu (liqueur-mère) — après y avoir dissous à chaud une quantité supplémentaire du dérivé de l'aniline souillé — au procédé de purification, on exécute le procédé de purification de la manière indiquée, et on répète encore au moins dix fois cette opération comprenant le recyclage de la liqueur-mère et le procédé de purification.

2. Procédé selon la revendication 1, caractérisé en ce que l'aniline à substituants électronégatifs répond à la formule générale (1)

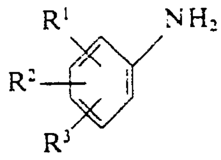

(1)

dans laquelle $R^1$ représente un atome d'hydrogène ou un radical méthoxy, éthoxy, méthyle ou éthyle, $R^2$ représente le groupe nitro, un atome de chlore ou de brome ou un radical cyano ou trifluorométhyle et $R^3$ représente un atome d'hydrogène, un radical méthoxy, éthoxy, méthyle ou éthyle ou un atome de chlore ou de brome, les symboles $R^1$, $R^2$ et $R^3$ pouvant avoir des significations identiques ou différentes.